# EUROPEAN PATENT SPECIFICATION

(11) **EP 2 759 537 B1**
(45) Date of publication and mention of the grant of the patent: **09.03.2016**
(21) Application number: 13781304.4
(22) Date of filing: 27.04.2013
(51) Int. Cl.: C07D 233/76, C07D 233/72, B01F 5/00, B01D 3/00, B01J 19/24

(54) **DEVICE AND METHOD FOR PREPARING 5-(2-(METHYLTHIO)ETHYL)HYDANTOIN**
VORRICHTUNG UND VERFAHREN ZUR HERSTELLUNG VON 5-(2-(METHYLTHIO)ETHYL)HYDANTOIN
DISPOSITIF ET PROCÉDÉ DESTINÉS À LA PRÉPARATION DE 5-(2-(MÉTHYLTHIO)ÉTHYL)HYDANTOÏNE

(30) Priority: 28.04.2012 CN 201210130404
(43) Date of publication of application: 30.07.2014
(73) Proprietor: Chongqing Unisplendour Tianhua Methionine Co., Ltd, Fengdu County, Chongqing 408200 (CN)
(72) Inventor: LUO, Yucheng, Chongqing 408200 (CN); HUANG, Baoyu, Chongqing 408200 (CN); YANG, Ping, Chongqing 408200 (CN); YANG, Yong, Chongqing 408200 (CN); CHANG, Kerang, Chongqing 408200 (CN); WEI, Tianlu, Chongqing 408200 (CN); LIU, Yaling, Chongqing 408200 (CN); LIU, Daowei, Chongqing 408200 (CN); ZHU, Nengjun, Chongqing 408200 (CN)
(74) Representative: ABG Patentes, S.L.
(86) International application number: PCT/CN2013/074858
(87) International publication number: WO 2013/159741

(56) References cited:
- AU-B2- 667 303
- CN-A- 1 160 043
- CN-A- 102 399 177
- CN-A- 102 659 684
- GB-A- 1 108 926
- US-A- 5 770 769

## Description

### FIELD OF THE INVENTION

The present invention belongs to the field of chemical industry, and relates to an improved device and method for preparing a chemical product.

### BACKGROUND OF THE INVENTION

Methionine is one of the basic units for constituting protein and the only sulfur-containing amino acid among the essential amino acids. It is a raw material for the synthesis of a protein and cystine besides participating in transmethylation, metabolism of phosphorus and synthesis of adrenaline, choline and creatine in animal bodies. Methionine is widely applied in the fields of medicine, food, feedstuff, cosmetics and the like, and is most consumable as a feedstuff additive. However, currently almost all methionine in our country is imported. The domestic production is very low and far from meeting the needs.

Hydantoin, with the chemical name of 5-(2-(methylthio)ethyl)hydantoin, is a key intermediate for preparing DL-methionine. 5-(2-(methylthio)ethyl)hydantoin is hydrolyzed with an alkali to generate DL-methionine salt which is subsequently neutralized with an acid to obtain DL-methionine. At present, the methods for preparing 5-(2-(methylthio)ethyl)hydantoin at home and abroad comprise a one-step method and a two-step method. The one-step method refers to feeding acraldehyde, methyl mercaptan, cyanide and hydantoin-forming reagents together to prepare 5-(2-(methylthio)ethyl)hydantoin, and it is a heterogeneous reaction, low in reaction speed and prone to such side reactions as polymerization. The two-step method comprises first preparing 3-(methylthio)propionaldehyde (TPMA) from acraldehyde and methyl mercaptan, and reacting 3-(methylthio)propionaldehyde, cyanide, carbon dioxide and ammonia to obtain 5-(2-(methylthio)ethyl)hydantoin. In industrial production using the two-step method, synthetic quality of 3-(methylthio)propionaldehyde in the first step can be well controlled, however in the second step, the reaction for synthesis of 5-(2-(methylthio)ethyl)hydantoin is performed at high temperature and high pressure, thus it is difficult to control the reaction conditions and liable to cause side reactions such as degradation of organic raw materials and polymerization, so as to induce decrease in yield of 5-(2-(methylthio)ethyl)hydantoin and instability of process.

### DESCRIPTION OF THE INVENTION

In view of this, the object of the present invention is to provide a method for preparing 5-(2-(methylthio)ethyl)hydantoin, which enables easy control on reaction temperature, well control of byproduct generation and improvement in the quality and yield of 5-(2-(methylthio)ethyl)hydantoin.

In order to achieve the above purpose, the present invention describes a device for preparing 5-(2-(methylthio)ethyl)hydantoin, comprising three stages of static mixing reactors connected in series and a stripping column, wherein a discharge port of a first-stage static mixing reactor is in communication with a feed port of a second-stage static mixing reactor, a discharge port of the second-stage static mixing reactor is in communication with a feed port of a third-stage static mixing reactor, and a discharge port of the third-stage static mixing reactor is in communication with a feed port of the stripping column.

Preferably, the top of the stripping column is provided with a gas-liquid separator, such as a cyclone separator. The device can well remove the liquid carried in the recycled gas.

Preferably, a relief valve is further arranged in the passage from the discharge port of the third-stage static mixing reactor to the feed port of the stripping column. The pressure of the high-pressure fluid flowing out from the discharge port of the third-stage static mixing reactor can be conveniently released to a certain extent using the device, and then fed to the stripping column.

The technical solution provided by the present invention is a method for preparing 5-(2-(methylthio)ethyl)hydantoin using the above-described device, comprising the following steps: feeding 3-(methylthio)propionaldehyde, sodium cyanide, and an aqueous solution of excessive carbon dioxide and ammonia to the three stages of static mixing reactors connected in series to undergo a continuous three-stage reaction, the pressure in the three stages of static mixing reactors connected in series being controlled to be 1.4-2.6 MPa, wherein the temperature in the first-stage static mixing reactor rising with a gradient from 40 °C to 80 °C, the temperature in the second-stage static mixing reactor rising with a gradient from 80 °C to 120 °C, and the temperature in the third-stage static mixing reactor rising with a gradient from 120 °C to 160 °C; and feeding the effluent from the discharge port of the third-stage static mixing reactor to the atmospheric pressure steam stripping column for separating and recovering un-reacted carbon dioxide and ammonia, the effluent from the bottom of the column being an aqueous solution of 5-(2-(methylthio)ethyl)hydantoin, and the mixed gas discharged from the top of the column being used for recovering and preparing the aqueous solution of carbon dioxide and ammonia and being recycled in the preparation of 5-(2-(methylthio)ethyl)hydantoin.

The reaction equation involved is

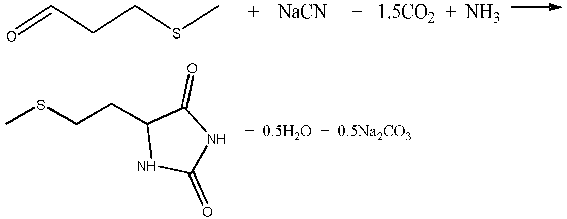

Preferably, the pressure in the three stages of static mixing reactors connected in series being controlled to be 1.6-1.8 MPa, and the total residence time of the reaction is 16-20 min. Under the preferred conditions, high reaction speed, short period, less byproducts and high 5-(2-(methylthio)ethyl)hydantoin yield can be achieved, and the requirements for the materials of the device and the connection parts are not high.

Preferably, the aqueous solution of carbon dioxide and ammonia, sodium cyanide, and 3-(methylthio)propionaldehyde are sequentially fed, i.e. sodium cyanide is first added to the aqueous solution of carbon dioxide and ammonia, and then 3-(methylthio)propionaldehyde is added. If 3-(methylthio)propionaldehyde is first added to the aqueous solution of carbon dioxide and ammonia, the obtained mixture has the risk of polymerization and it is liable to cause decrease in 5-(2-(methylthio)ethyl)hydantoin yield or process fluctuation and introduction of impurities to the product.

Preferably, 3-(methylthio)propionaldehyde and sodium cyanide are fed in a molar ratio of 1:1.02-1.03; in the aqueous solution of carbon dioxide and ammonia, carbon dioxide is not less than 12% w/v, ammonia is not less than 8% w/v, the molar ratio of carbon dioxide to ammonia is 1:1.5-1.9; and carbon dioxide and 3-(methylthio)propionaldehyde are fed in a molar ratio of 1.8-2.2:1. Under the above preferred conditions, 3-(methylthio)propionaldehyde reacts completely and is not prone to formation of polymer, so as to enable less byproducts and high yield of 5-(2-(methylthio)ethyl)hydantoin.

Preferably, the feeding ratio of 3-(methylthio)propionaldehyde to sodium cyanide is controlled by using a mass flowmeter. The feeding ratio of 3-(methylthio)propionaldehyde to sodium cyanide has a large influence on the quality and yield of 5-(2-(methylthio)ethyl)hydantoin. The existing technology adopts volumetric flowmeter to control the feeding ratio of both, which easily brings about feeding error due to volume change induced by temperature change. Serious fluctuation of feeding ratio in industrial production would lead to frequent interruption of production and low continuous production level. By replacing the volumetric flowmeter with a mass flowmeter, the feeding ratio of 3-(methylthio)propionaldehyde to sodium cyanide can be controlled accurately, so as to reduce generation of byproducts as much as possible, increase yield of 5-(2-(methylthio)ethyl)hydantoin, improve continuous production level, and reduce production cost.

Preferably, steam at a pressure of 0.2-0.8 Mpa is introduced into the steam stripping column. If the pressure of the water vapor is too high, the gas recycled from the top of the column will carry much liquid, which would result in loss of partial 5-(2-(methylthio)ethyl)hydantoin and decrease in 5-(2-(methylthio)ethyl)hydantoin yield; moreover, it will lead to poor quality of the aqueous solution of carbon dioxide and ammonia that is prepared from the recycled gas, thereby affecting subsequent preparation of 5-(2-(methylthio)ethyl)hydantoin. On the other hand, if the pressure of the water vapor is too low, the ammonium dissolved in the solution cannot be recycled sufficiently. Under the preferred conditions, carbon dioxide and ammonia can be recycled sufficiently and loss of 5-(2-(methylthio)ethyl)hydantoin is substantially avoided.

Preferably, the effluent from the discharge port of the third-stage static mixing reactor is subjected to pressure release with the relief valve to 0.8-1.0 MPa and fed to the stripping column. If the reaction mixture is fed to the atmospheric pressure stripping column in a high-temperature and high-pressure form, corrosion to the column body will readily occur. On the other hand, if the reaction mixture is fed to the atmospheric pressure stripping column after proper pressure release, it can not only allow the stripping operation with water vapor to proceed well, but also reduce corrosion of the column body and prolong service life of the equipment.

The advantageous effects of the present invention comprise: by using the three stages of static mixing reactors connected in series instead of the single reactor in the prior art to synthesize 5-(2-(methylthio)ethyl)hydantoin, the present invention can easily achieve gradient temperature rising, high-efficiency mixing, and complete reaction, and it not only realizes high reaction speed but also well controls generation of byproducts and improves the quality and yield of 5-(2-(methylthio)ethyl)hydantoin. In addition, the present invention employs atmospheric pressure water steam stripping column to separate and recycle un-reacted carbon dioxide and ammonia contained in the reaction mixture, without introducing new impurities, so that not only convenient and efficient refinement of 5-(2-(methylthio)ethyl)hydantoin is achieved, but also the mixed gas of water vapor, carbon dioxide and ammonia gas discharged from the top of the column can be used for recovering and preparing the aqueous solution of carbon dioxide and ammonia and be recycled in the preparation of 5-(2-(methylthio)ethyl)hydantoin. The preparation method of the present invention is applicable to large-scale continuous industrial production, and have good application prospect.

### DESCRIPTIONS OF THE FIGURES

In order to make the object, technical solutions and advantages of the present invention clearer, the present invention is further depicted in detail in combination with the following figure, wherein:

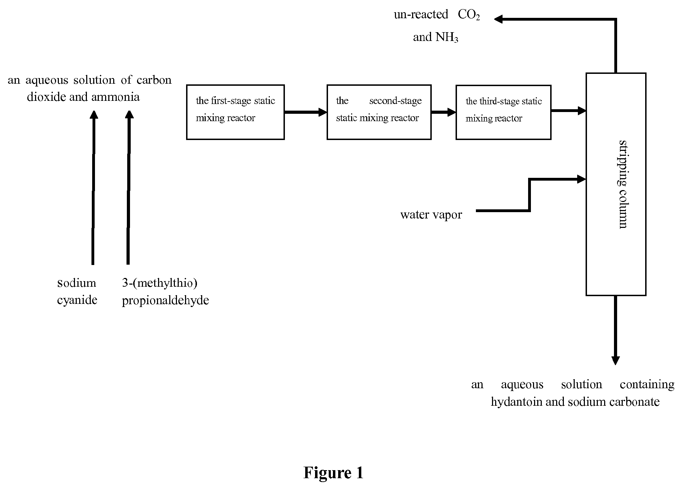

Fig. 1 shows a schematic diagram of the device used and the process flow of the method of the present invention.

### DESCRIPTION OF THE PREFERRED EMBODIMENTS

With reference to the figure, preferred examples of the present invention are elaborated.

Fig. 1 shows a schematic diagram of the device described in the present invention. The device for preparing 5-(2-(methylthio)ethyl)hydantoin comprises three stages of static mixing reactors connected in series and a stripping column, wherein a discharge port of a first-stage static mixing reactor is in communication with a feed port of a second-stage static mixing reactor, a discharge port of the second-stage static mixing reactor is in communication with a feed port of a third-stage static mixing reactor, and a discharge port of the third-stage static mixing reactor is in communication with a feed port of the stripping column.

As a preferred example of the device described in the present invention, a gas-liquid separator is further arranged on the top of the stripping column; and a relief valve is further arranged in the passage from the discharge port of the third-stage static mixing reactor to the feed port of the stripping column.

Fig. 1 shows a schematic diagram for the process flow of the method of the present invention. An aqueous solution of carbon dioxide and ammonia (containing 13% by mass of carbon dioxide and 8% by mass of ammonia), a sodium cyanide solution, and 3-(methylthio)propionaldehyde are continuously added to the first-stage static mixing reactor, wherein the aqueous solution of carbon dioxide and ammonia is directly pumped by a centrifuge pump, the molar ratio of carbon dioxide to 3-(methylthio)propionaldehyde is 2:1, the sodium cyanide solution and 3-(methylthio)propionaldehyde are delivered with a high-precision metering pump, and the feeding ratio of sodium cyanide to 3-(methylthio)propionaldehyde is controlled accurately with a mass flowmeter. The materials are mixed with high efficiency in the three stages of static mixing reactors connected in series and undergo a continuous three-stage reaction at a certain pressure and a temperature rising with gradient, wherein the temperature in the first-stage static mixing reactor rises with a gradient from 40 °C to 80 °C, the temperature in the second-stage static mixing reactor rises with a gradient from 80 °C to 120 °C, and the temperature in the third-stage static mixing reactor rises with a gradient from 120 °C to 160 °C. The effluent from the discharge port of the third-stage static mixing reactor is subjected to pressure release with the relief valve to 0.8-1.0 MPa and then fed to the stripping column to separate and recycle un-reacted carbon dioxide and ammonia. The top of the stripping column is provided with a gas-liquid separator. The top of the column has a pressure of atmospheric pressure and a temperature of 101 °C, and the interior of the column is introduced with water vapor at a pressure of 0.5 MPa. The mixed gas discharged from the top of the column is used for recovering and preparing the aqueous solution of carbon dioxide and ammonia and is recycled in the preparation of 5-(2-(methylthio)ethyl)hydantoin. The effluent from the bottom of the column is aqueous solution of 5-(2-(methylthio)ethyl)hydantoin. The qualified product is a light yellow, clear and transparent solution. The solution additionally contains sodium carbonate as a byproduct, and can be hydrolyzed directly with an alkali to prepare DL-methionine salt. Table 1 shows the specific conditions and results of examples.

**Table 1: Conditions for preparation of 5-(2-(methylthio)ethyl)hydantoin and results**

| Examples | NaCN/TPMA molar ratio | Reaction pressure (Mpa) | Total residence time of reaction (min) | Content of 5-(2-(methylthio)ethyl)hydantoin in product (%) | yield (%) | Product appearance |
|---|---|---|---|---|---|---|
| 1 | 1.055 | 1.6 | 16 | 10.36 | 73.25 | Yellow, oily material deposited |
| 2 | 1.050 | 1.6 | 16 | 11.43 | 80.63 | Orange, clear and transparent |
| 3 | 1.030 | 1.6 | 16 | 12.82 | 90.68 | Light yellow, clear and transparent |
| 4 | 1.025 | 1.6 | 16 | 12.99 | 93.72 | Light yellow, clear and transparent |
| 5 | 1.020 | 1.6 | 16 | 12.65 | 89.44 | Light yellow, clear and transparent |
| 6 | 1.000 | 1.6 | 16 | 11.41 | 80.63 | lemony yellow, turbid |
| 7 | 0.980 | 1.6 | 16 | 9.74 | 68.85 | lemony yellow, turbid |
| 8 | 1.021 | 1.6 | 6 | 9.59 | 69.44 | Light yellow, oily material deposited |
| 9 | 1.023 | 1.6 | 8 | 10.38 | 75.14 | Light yellow, oily material deposited |
| 10 | 1.022 | 1.6 | 10 | 11.31 | 81.89 | Light yellow, oily material deposited |
| 11 | 1.024 | 1.6 | 12 | 12.18 | 88.15 | Light yellow, clear and transparent |
| 12 | 1.025 | 1.6 | 16 | 13.01 | 94.20 | Light yellow, clear and transparent |
| 13 | 1.026 | 1.6 | 18 | 12.96 | 93.84 | Light yellow, clear and transparent |
| 14 | 1.027 | 1.6 | 20 | 12.86 | 93.10 | Light yellow, clear and transparent |
| 15 | 1.028 | 0.6 | 16 | 9.47 | 68.35 | Light yellow, oily material deposited |
| 16 | 1.028 | 0.9 | 16 | 10.38 | 74.93 | Light yellow, oily material deposited |
| 17 | 1.029 | 1.2 | 16 | 11.26 | 81.27 | Light yellow, oily material deposited |
| 18 | 1.030 | 1.4 | 16 | 12.25 | 88.45 | Light yellow, clear and transparent |
| 19 | 1.024 | 1.6 | 16 | 12.97 | 93.66 | Light yellow, clear and transparent |
| 20 | 1.025 | 2.0 | 16 | 13.11 | 94.65 | Light yellow, clear and transparent |
| 21 | 1.020 | 2.3 | 16 | 12.89 | 93.10 | Light yellow, clear and transparent |
| 22 | 1.025 | 2.6 | 16 | 13.09 | 94.52 | Light yellow, clear and transparent |
| 23 | 1.032 | 1.6 | 16 | 12.66 | 90.12 | Light yellow, clear and transparent |
| 24 | 1.025 | 1.6 | 16 | 13.17 | 93.74 | Light yellow, clear and transparent |
| 25 | 1.024 | 1.6 | 16 | 13.06 | 93.02 | Light yellow, clear and transparent |
| 26 | 1.022 | 1.6 | 16 | 12.90 | 91.82 | Light yellow, clear and transparent |
| 27 | 1.027 | 1.6 | 16 | 13.27 | 94.49 | Light yellow, clear and transparent |
| 28 | 1.025 | 1.6 | 16 | 13.02 | 92.69 | Light yellow, clear and transparent |
| 29 | 1.030 | 1.6 | 16 | 12.73 | 90.64 | Light yellow, clear and transparent |
| 30 | 1.028 | 1.6 | 16 | 13.09 | 93.23 | Light yellow, clear and transparent |
| 31 | 1.022 | 1.8 | 20 | 12.31 | 88.02 | Light yellow, clear and transparent |
| 32 | 1.024 | 1.8 | 20 | 12.48 | 89.25 | Light yellow, clear and transparent |
| 33 | 1.025 | 1.8 | 20 | 13.01 | 94.25 | Light yellow, clear and transparent |
| 34 | 1.026 | 1.8 | 20 | 12.96 | 93.92 | Light yellow, clear and transparent |
| 35 | 1.027 | 1.8 | 20 | 12.86 | 93.28 | Light yellow, clear and transparent |
| 36* | 1.023 | 1.6 | 16 | 11.52 | 82.1 | Light yellow, oily material deposited |

| | | | | | | |
|---|---|---|---|---|---|---|
| Note: * refers to reaction performed merely using a one-stage static mixing reactor with the reaction temperature rising with gradient from 40 °C to 160 °C. | | | | | | |

It can be seen from examples 1-7 that, although the molar ratio of sodium cyanide to 3-(methylthio)propionaldehyde in reaction is 1, the feeding ratio of sodium cyanide to 3-(methylthio)propionaldehyde should be more than 1 since sodium cyanide decomposes into ammonia and sodium formate at high temperature. However, if sodium cyanide is excessive too much, 3-(methylthio)propionaldehyde tends to form dimers and trimers in alkaline ammonia bicarbonate solution so that the product contains deposited oily materials. Therefore, the charge molar ratio of sodium cyanide to 3-(methylthio)propionaldehyde is preferably 1.02-1.03. Referring to examples 8-14, if the residence time of reaction is too short, the temperature rise of the material from the feed port of the reactor to the outlet thereof is too fast, and it easily induces polymerization of 3-(methylthio)propionaldehyde and decomposition of sodium cyanide so as to decrease yield of 5-(2-(methylthio)ethyl)hydantoin. Hence, the residence time of the reaction is preferably 12-20 min, and more preferably 16-20 min. The reaction pressure in the synthesis of 5-(2-(methylthio)ethyl)hydantoin is controlled for the purpose of ensuring the whole reaction is performed in liquid phase to increase the reaction speed. With reference to examples 15-22, if the pressure is excessively low, carbon dioxide and ammonia may be present in gaseous form, so that the reaction speed is low, the reaction is incomplete in the residence time, the yield of 5-(2-(methylthio)ethyl)hydantoin is affected and 3-(methylthio)propionaldehyde tends to form dimers and trimers to make the product contain deposited oil materials; and an excessively high pressure will propose high requirements for the materials of regulating valve and the like in industrial production. Consequently, the reaction pressure is preferably 1.4-2.6 MPa, and more preferably 1.6-1.8 MPa, From examples 23-35, it can be seen that, when production is continuously carried out for 8 times and 5 times by using a mass flowmeter under the preferred conditions of feeding ratio, residence time of reaction and reaction pressure, stable feeding ratio of sodium cyanide to 3-(methylthio)propionaldehyde, high reaction speed, less byproducts, high 5-(2-(methylthio)ethyl)hydantoin yield, good process stability and high continuous production level are achieved.

The present invention compares the reactions effects of the three stages of static mixing reactors connected in series and a one-stage static mixing reactor under the same conditions (example 36). The result shows that, the reaction preformed using a single static mixing reactor at a temperature rising with gradient from 40 to 160 °C is liable to cause side reactions such as degradation of organic raw materials and polymerization due to the temperature rising with larger gradient and the temperature not easy to control. Moreover, a single static mixing reactor cannot ensure adequate residence time of reaction, leading to incomplete reaction, lower 5-(2-(methylthio)ethyl)hydantoin yield (82.1%) and un-reacted oily 3-(methylthio)propionaldehyde appearing in the product.

The high-pressure effluent from the discharge port of the third-stage static mixing reactor is subjected to pressure release with the relief valve to 0.8-1.0 MPa and then fed to the stripping column. The un-reacted carbon dioxide gas and ammonia gas contained in the reaction mixture are recycled by flash evaporation with low-pressure water vapor of different pressures to realize refinement of 5-(2-(methylthio)ethyl)hydantoin, with the effects shown in tables 2-4.

**Table 2: Each component and content thereof before and after stripping with 0.5 MPa water vapor (kg/h)**

| Components | 0.5MPa water vapor | Feed pressure | Materials fed to the column | Liquid from the bottom of the column | Gas from the top of the column |
|---|---|---|---|---|---|
| CO₂ | - | 0.8 MPa | 315.6 | - | 315.6 |
| NH₃ | - | 0.8 MPa | 352.1 | 48.4 | 303.7 |
| 5-(2-(methylt hio)ethyl)hydan toin | - | 0.8 MPa | 1511.5 | 1511.5 | - |
| Na₂CO₃ | - | 0.8 MPa | 516.0 | 516.0 | - |
| H₂O | 976.3 | - | 7253.3 | 7620 | 609.6 |
| Total mass flow | 976.3 | - | 9948.5 | 9695.9 | 1228.9 |

**Table 3: Each component and content thereof before and after stripping with 0.2 MPa water vapor (kg/h)**

| Components | 0.2 MPa water vapor | Feed pressure | Materials fed to the column | Liquid from the bottom of the column | Gas from the top of the column |
|---|---|---|---|---|---|
| CO₂ | - | 0.8 MPa | 315.6 | - | 315.6 |
| NH₃ | - | 0.8 MPa | 352.1 | 68.4 | 283.7 |
| 5-(2-(methy lthio)ethyl)hy dantoin | - | 0.8 MPa | 1511.5 | 1511.5 | - |
| Na₂CO₃ | - | 0.8 MPa | 516.0 | 516.0 | - |
| H₂O | 976.3 | - | 7253.3 | 7670.2 | 559.4 |
| Total mass flow | 976.3 | - | 9948.5 | 9766.1 | 1158.7 |

**Table 4: Each component and content thereof before and after stripping with 0.8 MPa water vapor (kg/h)**

| Components | 0.8 MPa water vapor | Feed pressure | Materials fed to the column | Liquid from the bottom of the column | Gas from the top of the column |
|---|---|---|---|---|---|
| CO₂ | - | 1.0 MPa | 315.6 | - | 315.6 |
| NH₃ | - | 1.0 MPa | 352.1 | 48.4 | 303.7 |
| 5-(2-(methylt hio)ethyl)hydan toin | - | 1.0 MPa | 1511.5 | 1503.3 | 8.2 |
| Na₂CO₃ | - | 1.0 MPa | 516.0 | 509.2 | 6.8 |
| H₂O | 976.3 | - | 7253.3 | 7521.1 | 708.5 |
| Total mass flow | 976.3 | - | 9948.5 | 9582 | 1342.8 |

Finally, it needs to note that the above examples are merely for explaining rather than limiting the technical solutions of the present invention. Although the present invention has been depicted with reference to the preferred examples of the present invention, a person skilled in the art should understand that there are a variety of variants of the invention in form and detail that fall within the scope defined by the claims attached.

## Claims

1. A method for preparing 5-(2-(methylthio)ethyl)hydantoin by using a device , said device comprising three stages of static mixing reactors connected in series and a stripping column, wherein a discharge port of a first-stage static mixing reactor is in communication with a feed port of a second-stage static mixing reactor, a discharge port of the second-stage static mixing reactor is in communication with a feed port of a third-stage static mixing reactor, and a discharge port of the third-stage static mixing reactor is in communication with a feed port of the stripping column,
and said method comprising feeding 3-(methylthio)propionaldehyde, sodium cyanide, and an aqueous solution of excessive carbon dioxide and ammonia to the three stages of static mixing reactors connected in series to undergo a continuous three-stage reaction, the pressure in the three stages of static mixing reactors connected in series being controlled to be 1.4-2.6 MPa, wherein the temperature in the first-stage static mixing reactor rising with a gradient from 40 °C to 80 °C, the temperature in the second-stage static mixing reactor rising with a gradient from 80 °C to 120 °C, and the temperature in the third-stage static mixing reactor rising with a gradient from 120 °C to 160 °C; and feeding the effluent from the discharge port of the third-stage static mixing reactor to the atmospheric pressure steam stripping column for separating and recovering un-reacted carbon dioxide and ammonia, the effluent from the bottom of the column being an aqueous solution of 5-(2-(methylthio)ethyl)hydantoin, and the mixed gas discharged from the top of the column being used for recovering and preparing the aqueous solution of carbon dioxide and ammonia and being recycled in the preparation of 5-(2-(methylthio)ethyl)hydantoin.

2. The method according to claim 1, wherein the pressure in the three stages of static mixing reactors connected in series is controlled to be 1.6-1.8 MPa, and the total residence time of the reaction is 16-20 min.

3. The method according to claim 1, wherein the aqueous solution of carbon dioxide and ammonia, sodium cyanide, and 3-(methylthio)propionaldehyde are sequentially fed.

4. The method according to claim 1, wherein 3-(methylthio)propionaldehyde and sodium cyanide are fed in a molar ratio of 1:1.02-1.03; in the aqueous solution of carbon dioxide and ammonia, carbon dioxide is not less than 12% w/v, ammonia is not less than 8% w/v, the molar ratio of carbon dioxide to ammonia is 1:1.5-1.9; and carbon dioxide and 3-(methylthio)propionaldehyde are fed in a molar ratio of 1.8-2.2:1.

5. The method according to claim 1 or 4, wherein the feeding ratio of 3-(methylthio)propionaldehyde to sodium cyanide is controlled by using a mass flowmeter.

6. The method according to claim 1, wherein steam at a pressure of 0.2-0.8 MPa is introduced into the steam stripping column.

7. The method according to claim 1 or 6, wherein the effluent from the discharge port of the third-stage static mixing reactor is subjected to pressure release with the relief valve to 0.8-1.0 MPa and then fed to the stripping column.

## Patentansprüche

1. Verfahren zum Aufbereiten von 5-(2-(methylthio)ethyl)hydantoin unter Verwendung einer Vorrichtung, die Vorrichtung umfassend drei Stufen von in Serie verbundenen, statischen Mischreaktoren und eine Strippkolonne, wobei eine Abführöffnung eines statischen Mischreaktors der ersten Stufe in Verbindung mit einer Zuführöffnung eines statischen Mischreaktors der zweiten Stufe steht, eine Abführöffnung des statischen Mischreaktors der zweiten Stufe in Verbindung mit einer Zuführöffnung eines statischen Mischreaktors der dritten Stufe steht, und eine Abführöffnung des statischen Mischreaktors der dritten Stufe in Verbindung mit einer Zuführöffnung der Strippkolonne steht,
und das Verfahren umfassend Zuführen von 3-(methylthio)propionaldehyd, Natriumzyanid und einer wässrigen Lösung aus überschüssigen Kohlendioxid und Ammoniak an die drei Stufen von in Serie verbundenen, statischen Mischreaktoren um eine kontinuierliche Dreistufenreaktion durchzulaufen, der Druck in den drei Stufen von in Serie verbundenen, statischen Mischreaktoren wird gesteuert, um 1,4 - 2,6 MPa zu sein, wobei die Temperatur in dem statischen Mischreaktor der ersten Stufe mit einem Gradienten von 40 °C auf 80 °C ansteigt, die Temperatur in dem statischen Mischreaktor der zweiten Stufe mit einem Gradienten von 80 °C auf 120 °C ansteigt, und die Temperatur in dem statischen Mischreaktor der dritten Stufe mit einem Gradienten von 120 °C auf 160 °C ansteigt; und Zuführen des Abflusses von der Abführöffnung des statischen Mischreaktors der dritten Stufe zu der Atmosphärendruckdampfstrippkolonne zum Trennen und Rückgewinnen von nichtreagierten Kohlendioxid und Ammoniak, der Abfluss von der Unterseite der Kolonne ist eine wässrige Lösung aus 5-(2-(methylthio)ethyl)hydantoin, und das gemischte Gas, das von der Oberseite der Kolonne abgelassen wird, wird zum Rückgewinnen und Aufbereiten der wässrigen Lösung aus Kohlendioxid und Ammoniak verwendet und wird in der Aufbereitung von 5-(2-(methylthio)ethyl)hydantoin gewonnen.

2. Verfahren nach Anspruch 1, wobei der Druck in den drei Stufen von in Serie verbundenen, statischen Mischreaktoren gesteuert wird, um 1,6 - 1,8 MPa zu sein, und die gesamte Verweildauer der Reaktion 16 - 20 min beträgt.

3. Verfahren nach Anspruch 1, wobei die wässrige Lösung aus Kohlendioxid und Ammoniak, Natriumzyanid und 3-(methylthio)propionaldehyd nacheinander zugeführt werden.

4. Verfahren nach Anspruch 1, wobei 3-(methylthio)propionaldehyd und Natriumzyanid mit einen molaren Verhältnis von 1 : 1,02 - 1,03 zugeführt werden; in der wässrigen Lösung aus Kohlendioxid und Ammoniak ist der Anteil von Kohlendioxid nicht weniger als 12% w/v, der Anteil von Ammoniak nicht weniger als 8% w/v, das molare Verhältnis von Kohlendioxid zu Ammoniak ist 1 : 1,5 - 1,9; und Kohlendioxid und 3-(methylthio)propionaldehyd werden mit einem molaren Verhältnis von 1,8 - 2,2 :1 zugeführt.

5. Verfahren nach Anspruch 1 oder 4, wobei das Zuführverhältnis von 3-(methylthio)propionaldehyd zu Natriumzyanid unter Verwendung eines Massendurchflussmessers gesteuert wird.

6. Verfahren nach Anspruch 1, wobei Dampf mit einem Druck von 0,2 - 0,8 MPa in die Dampfstrippkolonne eingeleitet wird.

7. Verfahren nach Anspruch 1 oder 6, wobei der Abfluss von der Abführöffnung des statischen Mischreaktors der dritten Stufe einer Druckentlastung mit dem Entlastungsventil auf 0,8 - 1,0 MPa unterzogen wird und dann der Strippkolonne zugeführt wird.

## Revendications

1. Procédé destiné à la préparation de 5-(2-(méthylthio)éthyle)hydantoïne à l'aide d'un dispositif, ledit dispositif comprenant trois étages de réacteurs de mélange statique connectés en série et une colonne d'entraînement, dans lequel un orifice d'évacuation d'un réacteur de mélange statique du premier étage est en communication avec un orifice d'alimentation d'un réacteur de mélange statique du deuxième étage, un orifice d'évacuation du réacteur de mélange statique du deuxième étage est en communication avec un orifice d'alimentation d'un réacteur de mélange statique du troisième étage, et un orifice d'évacuation du réacteur de mélange statique du troisième étage est en communication avec un orifice d'alimentation de la colonne d'entraînement,
et ledit procédé comprend l'alimentation de 3-(méthylthio)propionaldéhyde, de cyanure de sodium et d'une solution aqueuse de dioxyde de carbone et d'ammoniaque en excès dans les trois étages des réacteurs de mélange statique connectés en série pour subir une réaction continue en trois étages, la pression dans les trois étages des réacteurs de mélange statique connectés en série étant réglée pour se situer entre 1,4 et 2,6 MPa, la température dans le réacteur de mélange statique du premier étage s'élevant avec un gradient de 40 °C à 80 °C, la température dans le réacteur de mélange statique du deuxième étage s'élevant avec un gradient de 80 °C à 120 °C et la température dans le réacteur de mélange statique du troisième étage s'élevant avec un gradient de 120 °C à 160 °C ; et l'alimentation de l'effluent s'écoulant hors de l'orifice d'évacuation du réacteur de mélange statique du troisième étage dans la colonne d'entraînement à la vapeur d'eau à pression atmosphérique pour la séparation et la récupération du dioxyde de carbone et de l'ammoniaque qui n'ont pas réagi, l'effluent s'écoulant hors du fond de la colonne étant une solution aqueuse de 5-(2-(méthylthio)éthyl)hydantoïne, et le gaz mixte évacué de la tête de la colonne étant utilisé pour la récupération et la préparation d'une solution aqueuse de dioxyde de carbone et d'ammoniaque et étant recyclé dans la préparation de 5-(2-(méthylthio)éthyl)hydantoïne.

2. Procédé selon la revendication 1, dans lequel la pression dans les trois étages des réacteurs de mélange statique connectés en série est réglée pour se situer entre 1,6 et 1,8 MPa, et le temps de séjour total de la réaction est de 16 à 20 min.

3. Procédé selon la revendication 1, dans lequel la solution aqueuse de dioxyde de carbone et d'ammoniaque, le cyanure de sodium et le 3-(méthylthio)propionaldéhyde sont alimentés séquentiellement.

4. Procédé selon la revendication 1, dans lequel le 3-(méthylthio)propionaldéhyde et le cyanure de sodium sont alimentés selon un rapport molaire de 1:1,02 à 1,03 ; dans la solution aqueuse de dioxyde de carbone et d'ammoniaque, la teneur en dioxyde de carbone n'est pas inférieure à 12 % poids/volume, la teneur en ammoniaque n'est pas inférieure à 8 % poids/volume, le rapport molaire du dioxyde de carbone à l'ammoniaque est de 1:1,5 à 1,9 ; et le dioxyde de carbone et le 3-(méthylthio)propionaldéhyde sont alimentés selon un rapport molaire de 1,8 à 2,2:1.

5. Procédé selon la revendication 1 ou 4, dans lequel le rapport d'alimentation du 3-(méthylthio)propionaldéhyde au cyanure de sodium est réglé à l'aide d'un débitmètre massique.

6. Procédé selon la revendication 1, dans lequel de la vapeur d'eau à une pression de 0,2 à 0,8 MPa est introduite dans la colonne d'entraînement à la vapeur d'eau.

7. Procédé selon la revendication 1 ou 6, dans lequel l'effluent s'écoulant hors de l'orifice d'évacuation du réacteur de mélange statique du troisième étage est réduit en pression à une valeur comprise entre 0,8 et 1,0 MPa à l'aide de la soupape de décharge puis alimenté dans la colonne d'entraînement.
